# EUROPEAN PATENT SPECIFICATION

(11) **EP 1 147 205 B1**
(45) Date of publication and mention of the grant of the patent: **17.06.2009**
(21) Application number: 99962556.9
(22) Date of filing: 31.12.1999
(51) Int. Cl.: C12N 15/79, C12N 1/21, A61K 48/00

(54) **HIGH EFFICIENCY MAMMALIAN GENE EXPRESSION VECTORS THAT CONTAIN EXOGENOUS PROMOTER AND ENTIRE 5' UNTRANSLATED REGION IN THE UPSTREAM FROM START CODON FOR INHERENT GENE AS TRANSCRIPTION REGULATORY SITE**
HOCHEFFIZIENTE SÄUGETIER EXPRESSIONSVEKTOREN DIE EINEN EXOGENEN PROMOTOR UND EINE VOLLSTÄNDIGE 5'NICHT-TRANSLATIERTE REGION OBERHALB DES STARTCODONS ENTHALTEN ALS TRANSKRIPTIONSREGULATIONSSTÄTTE FÜR INHÄRENTE GENE
VECTEURS D'EXPRESSION MAMMALIENS A HAUTE PERFORMANCE PRESENTANT UN PROMOTEUR EXOGENE ET UNE SEQUENCE 5' COMPLETE NON TRADUITE EN AMONT DU CODON INITIATEUR POUR LE GENE INHERENT EN TANT QUE SITE REGULATEUR TRANSCRIPTIONNEL

(30) Priority: 31.12.1998 KR 19980063711
(43) Date of publication of application: 24.10.2001
(73) Proprietor: Viromed Co., Ltd, Kwanak-gu Seoul 151-818 (KR)
(72) Inventor: KIM, Sunyoung, Yongsan-ku, Seoul 140-030 (KR); LEE, Young, Joo, Sungbuk-ku, Seoul 136-100 (KR); YU, Seung, Shin, Songpa-ku, Seoul 138-050 (KR); KIM, Duk-Kyung, Songpa-ku, Seoul 138-768 (KR)
(74) Representative: Kiddle, Simon John
(86) International application number: PCT/KR1999/000855
(87) International publication number: WO 2000/040737

(56) References cited:
- EP-A- 0 255 320
- WO-A-89/01036
- CHAPMAN B S ET AL: "EFFECT OF INTRON A FROM HUMAN CYTOMEGALOVIRUS (TOWNE) IMMEDIATE- EARLY GENE ON HETEROLOGOUS EXPRESSION IN MAMMALIAN CELLS" NUCLEIC ACIDS RESEARCH, OXFORD UNIVERSITY PRESS, SURREY, GB, vol. 19, no. 14, 25 July 1991 (1991-07-25), pages 3979-3986, XP000569788 ISSN: 0305-1048
- DONG WAN KIM ET AL: "USE OF THE HUMAN ELONGATION FACTOR 1ALPHA PROMOTER AS A VERSATILE AND EFFICIENT EXPRESSION SYSTEM" GENE, ELSEVIER BIOMEDICAL PRESS. AMSTERDAM, NL, vol. 91, no. 91, 1990, pages 217-223, XP002952510 ISSN: 0378-1119
- WAKABAYASHI-ITO NORIKO ET AL: "Characterization of the regulatory elements in the promoter of the human elongation factor-1-alpha gene." JOURNAL OF BIOLOGICAL CHEMISTRY, vol. 269, no. 47, 1994, pages 29831-29837, XP002219802 ISSN: 0021-9258
- WIRTH MANFRED ET AL: "Construction of new expression vectors for mammalian cells using the immediate early enhancer of the human cytomegalovirus to increase expression from heterologous enhancer/promoters." GBF MONOGRAPHS, vol. 15, 1991, pages 49-52, XP001119558 International Workshop on Protein Glycosylation;Braunschweig, Germany; June 28-30, 1990, analytical aspects. 1991 VCH Verlagsgesellschaft mbH; VCH Publishers, Inc. Postfach 10 11 61, Boschstrasse 12, D-6940 Weinheim, Germany; Suite 909, 220 East 23rd Street, New York, New York 10010, USA
- AKRIGG A., WILKINS G. W., ORAM J. D.: 'The structure of the major immediate early gene of human cytomegalovirus strain AD169.' VIRUS RES. vol. 2, no. 2, March 1985, pages 107 - 121, XP001041077
- UETSUKI T., NAITO A., NAGATA S., KAZIRO.: 'Isolation and characterization of the human chromosomal gene for polypeptide chain elongation factor-1 alpha.' J. BIOL CHEM. vol. 264, no. 10, 05 April 1989, pages 5791 - 5798, XP002020945
- ADDISON C. L., HITT M., KUNSKEN D., GRAHAM.: 'Comparison of the human versus murine cytomegalovirus immediate early gene promoters for transgene expression by adenoviral vectors.' J. GEN. VIROL vol. 78, no. 7, July 1997, pages 1653 - 1661, XP002102028
- TAKEKOSHI M., MAEDA-TAKEKOSHI F., IHARA S., SAKUMA S., WATANABE Y.: 'Use of a glycoprotein gB promoter for expression of gene inserted into the human cytomegalovirus genome.' TOKAI J. EXP. CLIN. MED. vol. 23, no. 1, March 1998, pages 39 - 44, XP000996376
- Genbank, gb/M21295.1/HS5MIEG 02-AUG-1993 Human cytomegalovirus (HCMV) major immediate-early protein (IE) gene, complete cds. XP002905605
- Genbank, gb/J04617/HUMEF1A 07-NOV-1994 Human elongation factor EF-1-alpha gene, complete cds. XP002905606

## Description

### FIELD OF THE INVENTION

To express foreign genes temporarily or permanently in eukaryotic cells, recombinant expression vectors should contain the promoter region, polyadenylation signal region and eukaryotic selectable marker gene as well as genes for replication and selection in *E.coli.*

### BACKGROUND

Among the strategies for constructing recombinant vectors which can drive strong and stable expression of the gene of interest in animal cells, the representative method is to increase the level of gene expression by selecting efficient promoter or to induce self-replication of plasmid with SV40 replication origin by expressing SV40 T antigen. Recently, HCMV IE (human cytomegalovirus immediate early) promoter and human housekeeping gene EF1α promoter have been exploited to drive higl levels of gene expression in eukaryotic cells.

In addition, it has been reported that plasmid containing intron and untranslated exon sequences induce higher levels of gene expression than plasmid lacks intron sequences, so recombinant vectors with exogenous intron have been developed and used for stable and permanent gene expression.

Since the development of vectors which can induce strong and stable gene expression in eukaryotic cells is very important for success in gene therapy trials and efficient foreign gene expression in animal cells, novel mammalian gene expression vectors which can induce strong and stable gene expression in permanent or temporal manner are required.

Thus, the present inventors constructed eukaryotic gene expression vectors for stable and strong expression of foreign gene, based on the notices that vectors which have exogenous promoter and 5' untranslated region (5'UTR) containing entire exon and intron sequences in upstream from initiation codon for inherent gene induce efficient gene expression. Particularly, the vectors of the present invention are more efficient than HCMV IE or EF1 α expression vector without exon or intron in vivo. In addition, chimeric vector which contain enhancer of HCMV IE gene upstream of EF1 α promoter and its 5'UTR regiong can induce high levels of gene expression.

### SUMMARY OF THE INVENTION

It is an object of this invention to provide mammalian gene expression vector containing exogenous regulatory element for driving strong and stable gene expression in temporarily or permanently transfected various mammalian cells or in vivo.

It is a further object of this invention to provide stable mammalian gene expression vector for gene therapy using naked DNA.

it is an additional object of this invention to provide recombinant microorganism transformed with the said mammalian gene expression vector.

In general, present invention provides eukaryotic expression vector which contains multi-cloning, site, polyadenylation signal region of bovine growth hormone, SV40 promoter and replication origin, marker gene and *ColE*I replication origin. The vector contains additionally regulatory elements for gene expression, exogenous promoter, and entire 5' untranslated region (hereinafter referred to as 5' UTR) in upstream of initiation codon of inherent translation.

Particularly, this invention provides eukaryotic expression vector, which contains promoter/enhancer, exon 1, intron A and nucleotide sequences just before initiation codon ATG of exon 2 derived from HCMV IE as regulation element. In one preferred embodiment, this invention provides pCN vector comprising the regulatory element described by SEQ ID NO: 3.

This invention also provides minimized eukaryotic vector pCK, which is depleted unnecessary sequences and whose β-lactamase gene is replaced with kanamycine resistance gene.

In addition, this invention provides vectors with useful foreign gene in multi-cloning site of pCN or pCK. In one preferred embodiment, this invention provides pCN-VEGF or pCK-VEGF comprising VEGF (vascular endothelial growth factor) gene. However, it should not be taken to limit the scope of the invention.

In another aspect, this invention provides the *E.coli* strains transformed with the said vectors.

In further aspect, this invention provides gene therapy agents containing the said vectors active ingredient.

Further features of the present invention will appear hereinafter.

### GRIEF DESCRIPTION OF THE DRAWINGS

FIG. 1 is a flow diagram for the construction of plasmid pCN by inserting the fragment encompassing enhancer/promoter and 5'UTR derived from HCMV IE gene into pCDNA3.1
FIG. 2 is a flow diagram for the construction of plasmid pEF by inserting the fragment encompassing enhancer/promoter and 5'UTR derived from human EF1 α gene into pCDNA3.1

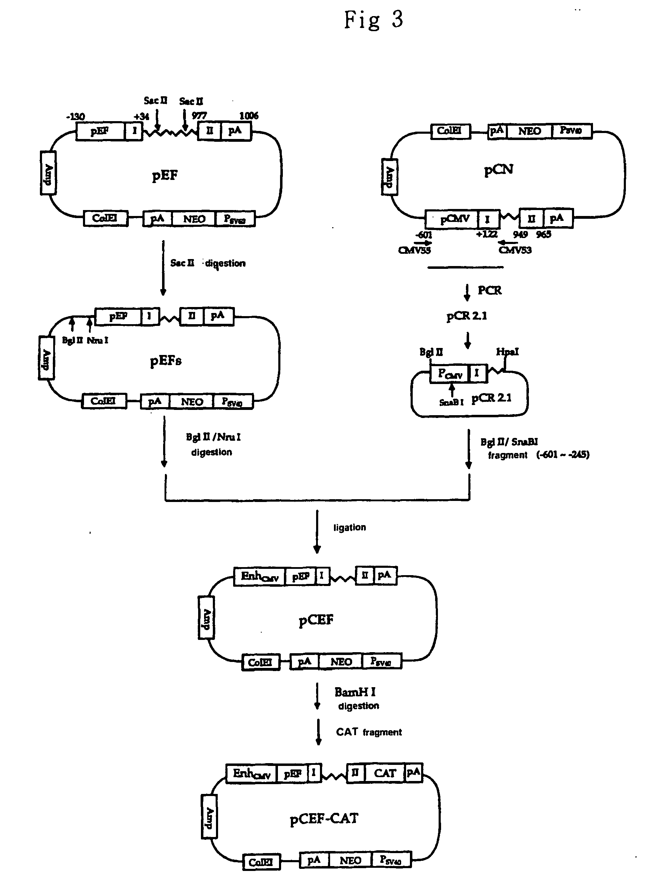

FIG. 3 is a flow diagram for the construction of pCEF by inserting the enhancer fragment derived from HCMV IE gene into pEFs vector containing promoter and partial intron sequence derived from EF1 α gene
FIG. 4 is a flow diagram for the construction of pCK vector,
FIG. 5 is a flow diagram for the construction of plasmid pCN/VEGF vector with VEGF121,
FIG. 6a to 6b is a flow diagram for the construction of plasmids pCK/VEGF165,
FIG. 7 is a flow diagram for the construction of pCP vector in which 5'UTR is deleted from pCK vector,
FIG. 8 is a flow diagram for the construction of plasmids pCDNA3.1, and pCN.
FIG. 9a to 9b is a flow diagram for the construction of plasmids pCP-gagrepresent the procedure for construction of pCP-gag/pol vector expressing *gag-pol* gene of MLV.
FIG. 10a to 10b is a flow diagram for the construction of plasmid pCH-env expressing *env* gene of MLV,

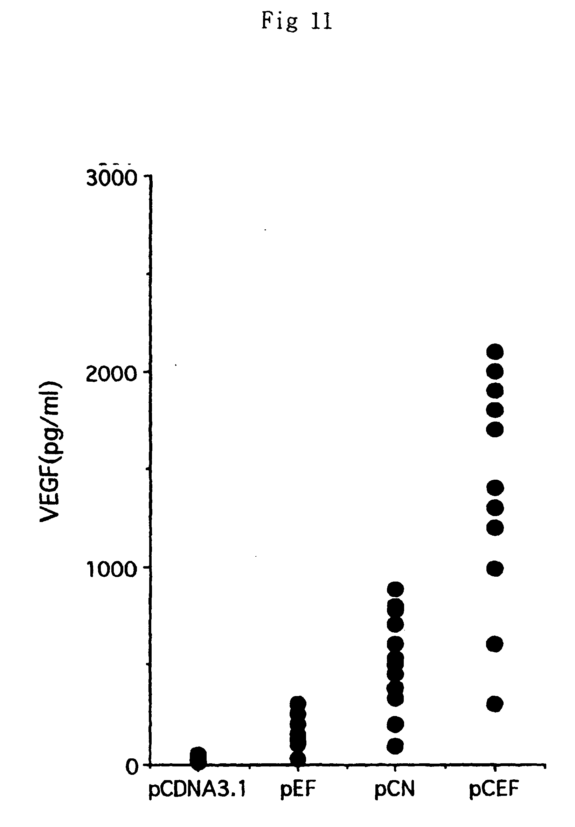

FIG. 11 is a histogram showing the gene expression levels of pCDNA3.1-VEGF, pEF-VEGF, pCN-VEGF and pCEF-VEGF vectors in transient transfection system,
FIG. 12 is a flow diagram of the construction plasmid pEF-CAT,

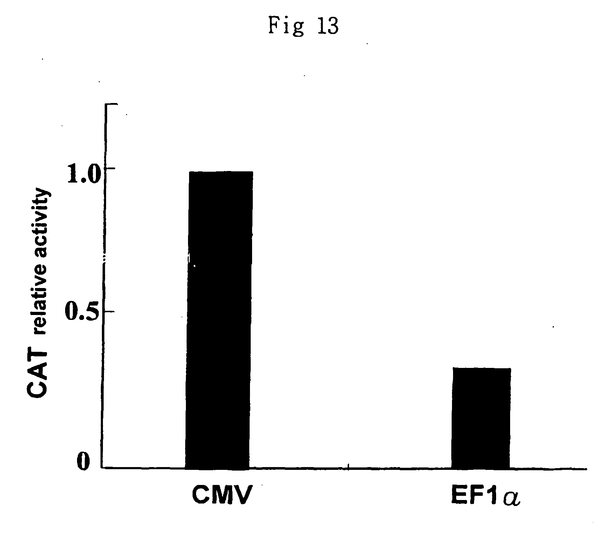

FIG. 13 is a histogram showing the CAT activities of HeLa cells transiently transfected pEF-CAT or pCN-CAT,
FIG. 14 is a histogram showing the CAT activities of HeLa cells stably transfected with pEF-CAT or pCN-CAT,
FIG. 15 is a histogram showing the time course of gene expression level of HeLa cells stably transfected with pEF-CAT or pCN-CAT vectors,
FIG. 16 is a histogram showing the gene expression level of the subclones obtained from HeLa cell populations stably trandsfected with pEF-CAT or pCN-CAT vectors,
FIG. 17 is a histogram showing the expression level of VEGF in the mouse muscle injected with pCN-VEGF vector,
FIG. 18a to 18b is a flow diagram of the construction of plasmids pCK-CAT and pCN-CAT,
FIG. 19 is a histogram showing CAT activities of C₂C₁₂ cells transfected with pCK-VEGF or pCP-VEGF vector,
FIG. 20 is a histogram showing VEGF levels of C₂C₁₂ cells transfected with pCK-VEGF165 or pCP-VEGF165 vector,
FIG. 21 is a histogram showing CAT activities of mouse muscle cells injected with pCK-CAT or pCP-CAT vector,
FIG. 22 is a histogram showing VEGF levels of mouse muscle cell injected with pCK-VEGF or pCP-VEGF vector,
FIG. 23 is a histogram showing the time course of gene expression in mouse muscle cells injected CAT gene,
FIG. 24 is a histogram showing the time course of VEGF expression in mouse muscle cells injected with pCK-VEGF165 vector,
FIG. 25 is a histogram showing dose-dependency of gene expression in mouse muscle injected with pCK-CAT,

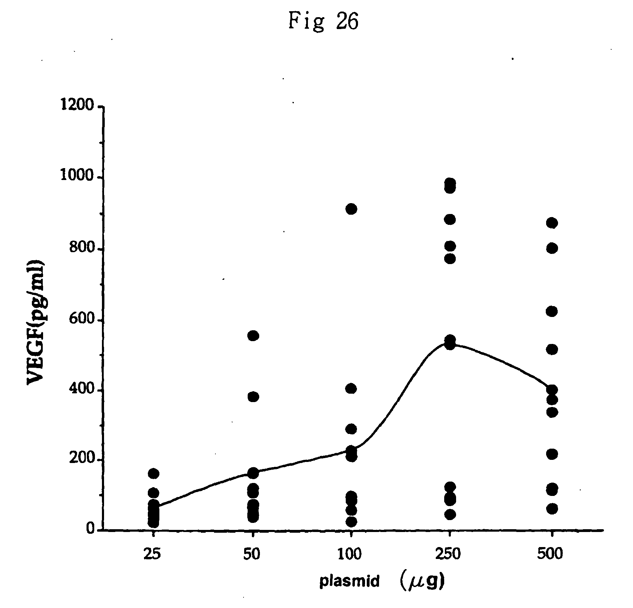

FIG. 26 is a histogram showing dose-dependency of gene expression in mouse muscle injected with pCK-VEGF165.

### DETAILED DESCRIPTION OF PREFERRED EMBODIMENTS

Hereinafter, the present invention is described in two cases of using HCMV IE promoter and, by way of a comparison with the present invention, using human EF1α promoter.

### 1. Expression vector derived from HCMV TE gene

### 1) construction cf pCN

It has been reported that intron A sequence derived from the HCMV IE gene increases the level of gene expression (Chapman et al., Nucl. Acids Res., 19:3979-3986, 1991). Based on this report, the present inventors constructed the eukaryotic expression vector, pCN vector, which contains promoter/enhancer and 5'UTR, namely from the start site of RNA transcription to nucleotide sequences just before initiation codon ATG (exon 1, intron A and partial exon 2) derived from HCMV IE gene as regulatory element and wherein foreign gene is expressed from spliced RNA.

The present inventors constructed a more efficient eukaryotic gene expression vector using pCDNA3.1 (Invitrogen) as initiation vector. DNA fragment containing enhancer/promoter and 5'UTR of HCMV IE gene was amplified by polymerase chain reaction (PCR), using pEQ276 vector(Biegalke et al., Virology, 183:381-385(1995)) as a template. The amplified DNA fragment was replaced with the HCMV enhancer/promoter fragment of the pCDNA3.1 vector, for construction of pCN vector. The backbone of pCN is therefore identical to pCDNA3.1, but the foreign gene inserted into the multi-cloning site is expressed from the spliced messenger RNA.

### 2) gene expression level from pCN

In order to investigate the effect of HCMV IE 5'UTR on the level of gene expression, plasmids pCDNA3.1-CAT, pCN-CAT, and pCIneo-CAT were constructed by inserting the CAT reporter gene into pCDNA3.1, pCN ,and pCIneo vector, respectively. 293T cells(DuBridge et al., Mol. Cell. Biol., 7:379-387, 1987) were transfected with these vectors, and the levels of CAT activity was measured in transfected cells. The CAT activity of cells transfected with pCN-CAT containing 5'UTR of HCMV IE, was about 60 times higher than that of cells transfected with vectors lacking 5'UTR.

In addition, as another indicator to confirm the gene expression level, packaging efficiency was measured. Particularly, *gag-pol* and *env* genes derived from MLV were inserted into the pCN vector respectively, and the resulting vectors together with retroviral vector MFG-CAT(Kim et al., J. Virol., 72:994-1004,1998) were transfected to 293T cells. 2 days after the viral supernatant was used to transduce NIH 3T3 cells. As a result, the CAT activity was higher than other conventional packaging vectors were used, suggesting pckaging constructs based on pCN provided gag/pol and env proteins efficiently. These results indicate that pCN vector of the present invention is very efficient expression vector.

### 2. Construction of a comparative expression vector derived from human EF1α gene

### 1) Construction of plasmid pEF

To complement the drawbacks of gene expression system using viral promoters, the present inventors cloned promoter and 5' UTR of human EF1α gene. Particularly, the chromosomal DNA fragment encompassing promoter, exon 1, intron A and nucleotide sequences just before ATG codon of exon 2 region of EF1α gene, was amplified by PCR using human placental RNA as a template. The PCR product was replaced with HCMV enhancer/promoter region of pCDNA3.1 vector for construction of pEF.

Thus, pEF vector contains EF1α promoter and entire 5'UTR (exon 1, intron A and partial exon 2) at upstream of the multi-cloning site. The gene expression of foreign gene inserted into the multi-cloning site is derived from spliced messenger RNA.

### 2) Gene expression level of pEF vector

In order to compare the gene expression efficiency of pCN and pEF vectors, the vectors were were transfected to HeLa cell and CAT activities were measured 2 days after transfection. As a result, in transient expression system, the pCN vector showed 2 fold higher CAT activity than pEF vector. However, when CAT activities were compared using stably transfected cells selected for a long time, the CAT activity of cells transfected with pEF showed higher levels gene expression that of cells transfected with pCN.

Namely, HCMV IE promoter drives transiently high-level gene expression in cell culture system, but drives low-level gene expression in stable system. However, in stable expression system, EF1α promoter derived from cellular gene works more efficiently than HCMV IE promoter derived from viral gene.

Therefore, pEF vector is preferable for a long-time stable gene expression whereas pCN vector is preferable for transient gene expression.

### 3. Construction of chimeric plasmid pCEF

### 1) construction of pCEF vector

To construct a novel expression vector with all advantages of both pCN and pEF, chimeric plasmid pCEF was constructed.

Concretely, enhancer of HCMV IE gene was inserted at the upstream of promoter region of pEF and promoter and intron of EF1α gene was maintained, allowed to drive high levels of gene expression both transiently and stably.

First, the unnecessary intron sequences from +57 to +409 position in about 900 bp-length intron of the pEF vector was depleted, and generating pEFs. The enhancer sequence of HCMV IE gene was amplified by PCR, and then inserted at upstream of EF1α promoter of the depleted, pEFs vector, generating the chimeric vector, pCEF.

### 2) gene expression level of pCEF vector

To compare the transcription efficiency of the chimeric vector with that of other known vectors, pCEF-CAT was constructed by inserting the CAT fragment into *BamH*I site of pCEF. pCN-CAT and pEF-CAT, were transfected to 293T cells, respectively and the CAT activities were measured 2 days after transfection. PCEF produced 5-fold, and 2-fold higher levels of CAT activity than pEF and pCN did, respectively.

### 4. Construction of pCK suitable for naked DNA gene therapy

### 1) construction of pCK

In order to make pCN better fit in the context of naked DNA gene therapy, we have modified backbone of pCN, eventually constructing pCK. In pCK, the β-lactamase gene was replaced with the gene conferring the resistance to kanamycin because residual ampicillin in final DNA solution might cause allergic reactions to some patients undergoing gene therapy trials. Furthermore, all possible nucleotide sequences unnecessary for a vector to function as a gene delivery vehicle were removed from the plasmid in order to minimize the size of the plasmid to 3.7 kb, resulting in a relatively high copy number in E. coli.

### 2) Gene expression level of pCK

pCK vector was expressed efficiently by *E.coli* and the safety was improved. As an embodiment of this vector, pCK-VEGF165 was constructed and the level of gene expression was measured *in vivo*/*in vitro.* As shown in the result, the gene expression level derived from the pCK was 30-fold higher than that from control vector pCP which lacking 5' UTR, suggested that the said pCK-VEGF165 vector could be used for the treatment of ischemic disease.

### 5. Use of pCK, pEF, pCK and pCEF vectors as gene therapy agents

In order to test the possibilities of using above pCN and pEF vectors as gene therapy agents, pCN-VEGF, pEF-VEGF and pCEF-VEGF vectors were constructed by inserting of VEGF gene into pCN, pEF and pCEF vectors, respectively. And then, the constructed vectors were transfected to mouse muscle cell lines C₂C₁₂ (ATCC CRL-1772), and the VEGF protein level was measured by ELISA. All of the above three vectors produced high levels of VEGF protein. Additionally, to investigate the protein expression level *in vivo,* the constructed vectors were injected into the anterior fibialis muscle of 2-week-old Balb/C male mouse, and the amount of VEGF protein produced was measured 2 days after injection. As a result, pCN-VEGF and pCEF-VEGF produced higher levels of VEGF activity than pCEF-VEGF in the injected muscle. These results suggested that pCN and pCEF vectors could drive high-level gene expression *in vivo and in vitro.*

As mentioned above, the pCK vector was produced in high copy number in E.coli and the safety improved. As an embodiment of this vector, pCK-VEGF165 was constructed and the level of gene expression was investigated *in vivo*/*in vitro.* In result, the expression level of the vector is more than 30 times as high as that of pCP vector without 5' UTR, and thus the said pCK-VEGF165 vector can be used for treatment of inchemic disease. As pre-clinical test for a new drug development, the time-course of protein production and the acute toxicity test were performed.

The E.coli transformant by pCN/VEGF vector was designated to 'Top10-pCN/VEGF' and deposited to Korean Culture Center of Microorganisms on Nov 19, 1998 (Accession NO: KFCC-11064).

The E.coli transformant by pEF/VEGF vector was designated to 'Top10-pEF/VEGF' and deposited to Korean Culture Center of Microorganisms on Nov 19, 1998 (Accession NO: KFCC-11063).

The E.coli transformant by pCK-VEGF165 vector was designated to 'Top10-pCK/VEGF165' and deposited to Korean Culture Center of Microorganisms on December 27, 1999 (Accession NO: KCCM-10179).

By all results, because of their abilities to drive high levels of gene expression, pCN, pEF, pCEF and pCK vectors can be applied for continuous expression of foreign gene, especially for naked DNA gene therapy *in vivo.* Specially, the use of backbone plasmid pCK is suitable for naked gene therapy because of the improved safety and the increased productivity of the vector.

### EXAMPLES

Practical and presently preferred embodiments of the present invention are illustrative as shown in the following Examples.

### Example 1: construction of pCN

To construct pCN, the full length of IE enhancer/promoter(hereinafter referred to as "P_{CMV}") of HCMV and its entire 5' untranslated region (5' UTR) was cloned by PCR. pEQ276 vector(Biegalke et al., Virology, 183:381-385(1995)) containing from the promoter to exon 5 of HCMV IE gene was used as a template and two synthetic oligonucleotides which were described by SEQ ID NO: 1 and NO: 2 were used as primers.

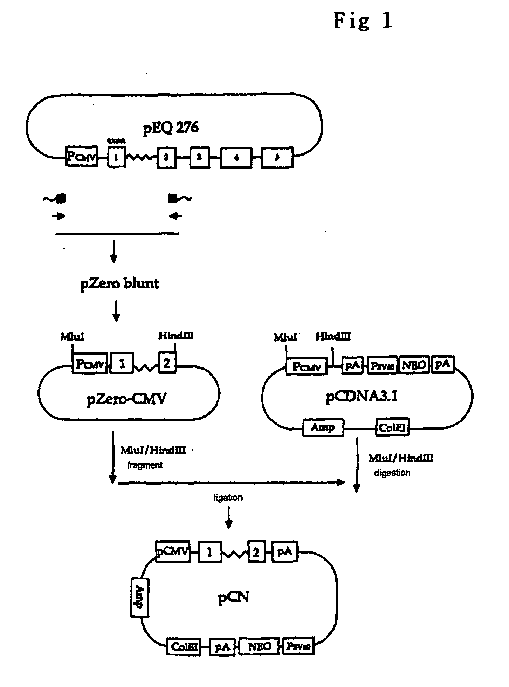

The amplified 1.6kb DNA fragment corresponded to the nucleotide sequence containing enhancer/promoter, exon 1, intron A and upstream sequence of initiation codon of exon 2 (SEQ. ID NO: 3). Nucleotide sequence from 1 to 599 corresponded to P_{CMV}, sequence from 600 to 720 did to exon 1, sequence from 721 to 1547 did to intron A and sequence from 1548 to 1564 did to upstream sequence of initiation codon of exon 2. The PCR product was cloned into pZeroBlunt vector(Invitrogen) to generate pZero-CMV. Then, the MluI-HindIII fragment containing P_{CMV} of pCDNA3.1 vector(Invitrogen) was replaced with the MluI/HindIII fragment of the resulting vector pZero-CMV, generating pCN vector(see FIG 1). Therefore the backbone of pCN is identical to pCDNA3.1 containing multi-cloning site, BGH(bovine growth hormone) polyadenylation signal region(hereinafter referred to as "pA"), SV40 promoter(hereinafter referred to as "PSV40") and replication origin, neomycin antibiotic gene(hereinafter referred to as "NEO") and ColE1 replication origin as well as P_{CMV}. pCN differs from pcDNA3.1 in which entire 5'UTR of HCMV IE(exon 1, intron A and partial exon 2) in upstream of the multi-cloning site, thus the foreign gene inserted into the multi-cloning site was expressed from spliced messenger RNA.

E.coli transformed by the said pCN vector was desingnated 'Top10-pCN' and deposited in Korean Culture Center of Microorganisms on july 20, 1998(Accession NO: KFCC-11045).

### Example 2: construction of pEF

To construct pEF, the promoter region of human EF1α gene and its 5'UTR was cloned by PCR using a pair of primers which were described by SEQ ID NO: 4 and NO: 5.

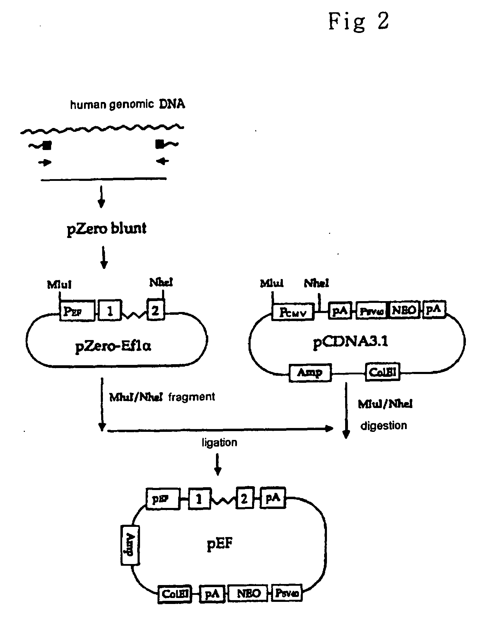

1.2 kb-length DNA fragment of PCR product contains enhancer/promoter(hereinafter referred to as "P_{EF}"), exon 1, intron A and upstream sequence of ATG codon of exon 2 derived from EF1α gene(SEQ ID NO: 6). The nucleotide sequence from 1 to 137 corresponded to P_{EF}, sequence from 138 to 158 did to exon 1, sequence from 159 to 1104 did to intron A and sequence from 1105 to 1136 did to upstream sequence of ATG codon of exon 2. The PCR product was cloned into pZeroBlunt vector generating pZero-EF1α. The MluI/NheI region of pCDNA3.1 vector was replaced with Mlu/NheI fragment prepared from the pZero-EF1α, generating pEF vector(see FIG 2). Because the backbone of pEF is identical to pCDNA3.1, pEF contains multi-cloning site, BGH pA, P_{SV}40 and replication origin, NEO and ColE1 replication origin. In addition, it contains P_{EF} and its 5' UTR of HCMV IE(exon 1, intron A and partial exon 2) at upstream of the multi-cloning site, thus the foreign gene inserted into the multi-cloning site was expressed from spliced messenger RNA.

### Example 3: construction of pCEF

It was observed that the pCN produced high levels of gene expression in transient transfection system, while the pEF produced high levels of gene expression in stable transfection system. So the inventors of this invention constructed a chimeric vector to take both advantages of the two vector system.

Namely, the chimeric vetor was constructed by inserting the enhancer region of HCMV gene into the 5' end of P_{EF} thus allowed to maintain the rest of the structure. The enhancer sequence of HCMV IE gene was cloned by PCR amplification of pEQ276 using a pair of primers described by SEQ ID NO: 7 and NO: 8.

The original pEF vector contains about 900 bp-length intron. To reduce the size of the intron, the nucleotide sequence from +57 to +409 position was removed by internal deletion using SacII restriction enzyme, generating pEFs. BglII-SnaBI HCMV enhancer fragment was inserted into NruI-BglII site of pEFs, generating pCEF.

### Example 4: construction of pCK

In order to make vector better fit in the context of naked DNA gene therapy, we have modified backbone of pCN, eventually constructing pCK. In pCK, the β-lactamase gene was replaced with the gene conferring the resistance to kanamycin because residual ampicillin in final DNA solution might cause allergic reactions to some patients undergoing gene therapy trials. Furthermore, all possible nucleotide sequences unnecessary for a vector to function as a gene delivery vehicle were removed from the plasmid in order to minimize the size of the plasmid, resulting in a relatively high copy number in *E.coli.* The nucleotide sequences encompassing promoter region and its 5' UTR, and BGHpA region of pCN vector was amplified by PCR using a pair of primers described by SEQ ID NO: 9 and NO: 10.

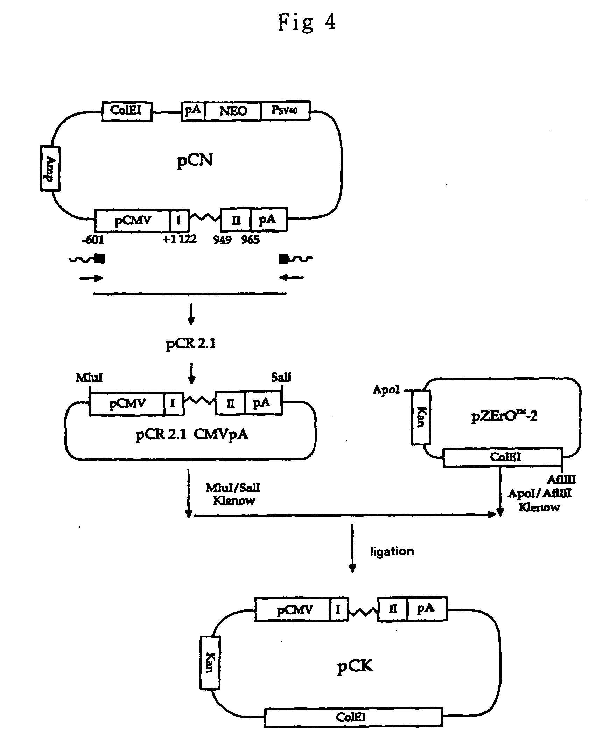

The DNA fragment was cloned into pCR2.1 vector(Invitrogen), generating pCR2.1CMVpA. The resulting vector was then treated with restriction enzyme and MluI/SalI, the fragment containing PCRed was purified. Then, the fragment containing kanamycin resistance gene and ColEI replication origin was isolated from pZero2.1 vector(Invitrogen) using ApoI and AflIII, religated with MluI/SalI fragment from pCR2.1CMVpA, constructing pCK vector(see FIG 4).

### Example 5: construction of pCN-VEGF

cDNA encoding human VEGF121 gene was cloned from total RNA prepared from human placenta by RT-PCR(reverse transcription PCR). PCR primers were described by SEQ ID NO: 11(sense primer) and NO: 12(antisense primer).

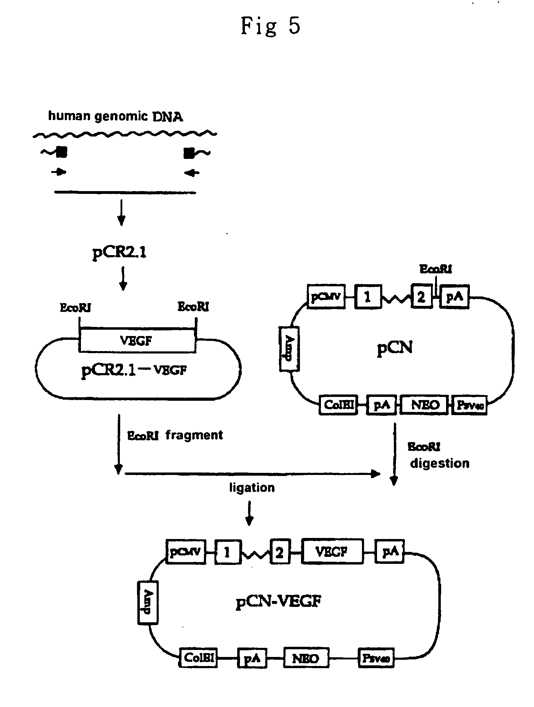

The RT-PCR product was cloned into pCR2.1 vector (Invitrogen) and EcoRI fragment of the resulting vector was inserted into *EcoR*I site of pCN vector, generating pCN-VEGF vector(see FIG 5).

### Example 6 : construction of pEF-VEGF

All the procedures were conducted according to Example 5, except that pCN vector was replaced with pEF vector.

### Example 7: construction of pCEF-VEGF

All the procedures were conducted according to Example 5, except that p CN vector was replaced with pCEF vector.

### Example 8: construction of pCK-VEGF

cDNA encoding VEGF165 was cloned from total RNA prepared from human vascular smooth muscle cells by RT-PCR. PCR primers were described by SEQ ID NO: 13 and NO: 14. The amplified cDNA was initially cloned into the pCR2.1 vector(Invitrogen), and its nucleotide sequence was continued by sequencing.

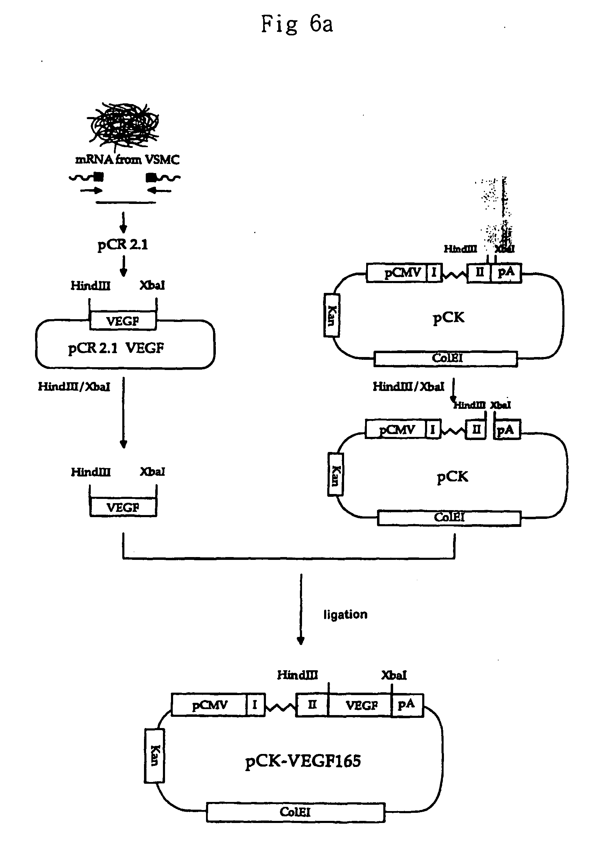

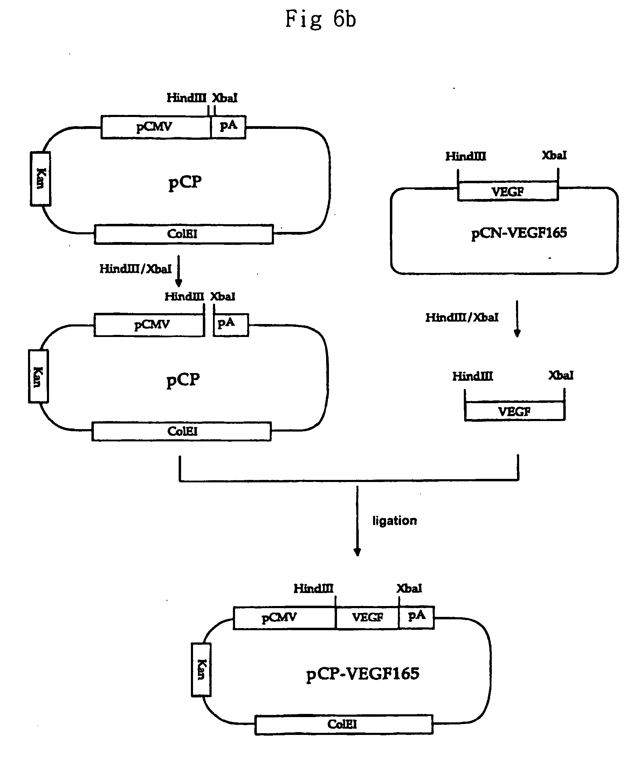

The *Hind*III/*Xba*I fragment containing VEGF165 from pCR2.1-VEGF165 was inserted into *Hind*III/*Xba*I site of pCK vector, generating pCK-VEGF165 (see FIG 6).

*E.coli* transformed by pCK-VEGF165 was designated 'Top10-pCK/VEGF165' and deposited to Korean Culture Center of Microorganisms on December 27, 1999(Accession NO: KCCM-10179).

### Example 9: construction of pCP and pCP-VEGF

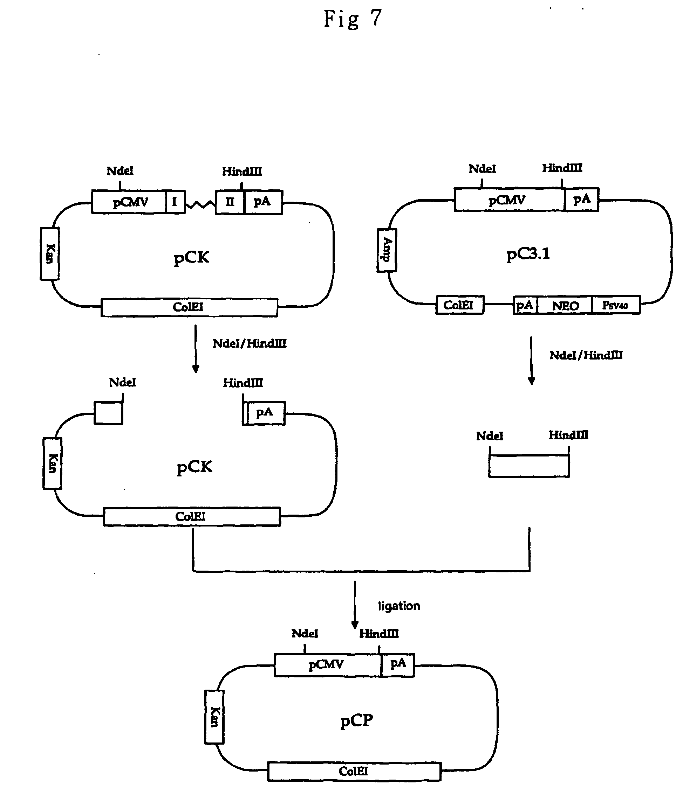

As a control plasmid, pCP whose backbone is identical to that of pCK except that it lacks the untranslated leader sequences of the major IE region of HCMV was constructed. The pCP was constructed by inserting the *Nde*I/*Hind*III fragment from pCDNA3.1 into *Nde*I/*Hind*III site of pCK(see FIG 7).

And then, pCP-VEGF165 vector was constructed by inserting the *Hind*III/*Xba*I fragment of pCK-VEGF165 containing VEGF165 gene into *Hind*III/*Xba*I site of pCP.

### Example 10: Effect of HCMV IE 5'UTR region on the levels of gene expression

### 1) construction of pCDNA3.1-CAT, pCIneo-CAT and pCN-CAT

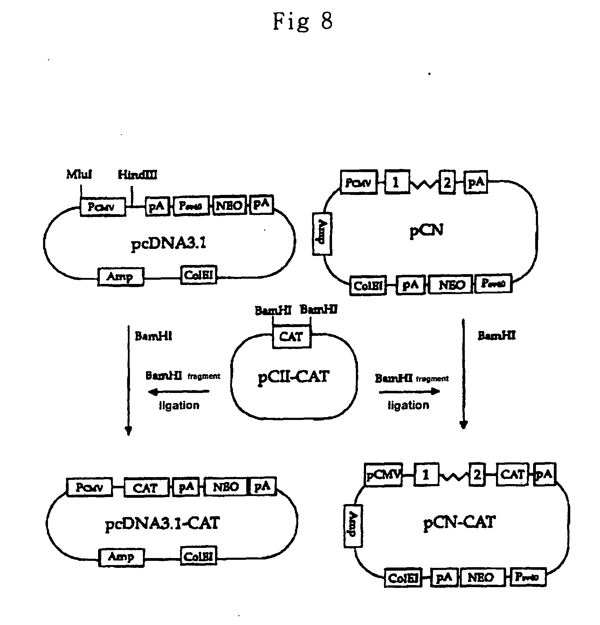

To compare the levels of gene expression from pCN with other commercially available vectors, we chose pCDNA3.1(Invitrogen) and pCIneo(Promega). pCDNA3.1 contains P_{CMV} alone, whereas pCIneo contains heterologous synthetic intron. pCDNA3.1-CAT, pCN-CAT and pCIneo-CAT were constructed through the insertion of CAT reporter gene into *Bam*HI site of each plasmid, respectively(see FIG 8).

### 2) comparison the level of gene expression

The pCN-CAT, pCDNA3.1-CAT and pCIneo-CAT vector, were transfected to 293T cells(DuBridge et al., Mol. Cell. Biol., 7:379-387, 1987). 2 days after transfection, protein extracts were prepared to measure the level of gene expression. As a result, as shown in table 1, the CAT activity from pCN-CAT containing 5' UTR was 60 fold higher than those from control vectors. These results suggest that the 5' UTR sequences attribute to increase the level of gene expression.

**Table 1**

| Effect of HCMV IE 5' UTR insertion on gene expression efficiency | |
|---|---|
| Vector | CAT activity |
| pCDNA3.1-CAT | 0.6(+/-0.1) |
| pCIneo-CAT | 0.7(+/-0.2) |
| pCN-CAT | 37.9(+/-2.0) |

### Example 11: Effect of pCN vector on virus packaging

In order to investigate the effect of the above constructed pCN vector on the gene expression efficiency and on retroviral packaging, retroviral vectors, gag-pol vector and env vector, were constructed, using the pCN vector as elementary backbone. To distinguish the marker from the inherent neomycin gene of retroviral vectors, the marker gene inserted into the above packaging vector was puromycin antibiotic gene for gag-pol vector and hygromycin antibiotic gene for env vector, respectively.

### 1) pCP-gag/pol vector construction

The pCP-gag/pol vector, which contained puromycin gene as marker for eukaryote and enhancer/promoter and 5' UTR sequence of HCMV IE gene as regulator, was constructed:

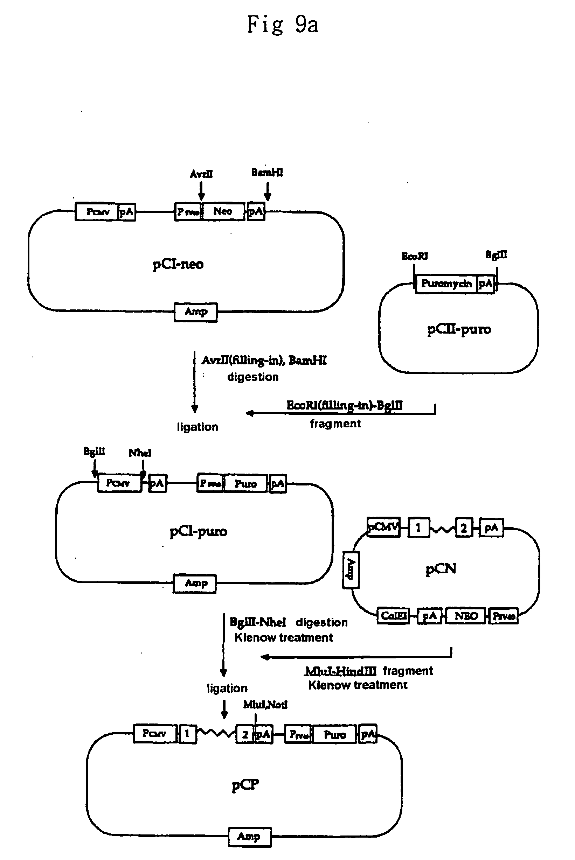

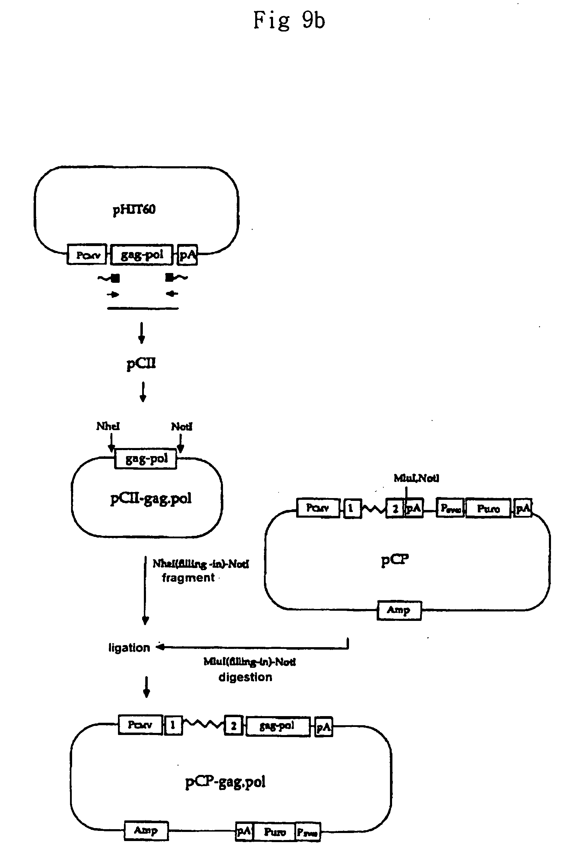

First, neo gene was deleted from the pCI-neo vector(Promega) and then pCI-puro vector was constructed through insertion of puromycin gene fragment separated from pCII-puro vector into the resulting vector(see FIG 9a). After the above pCI-puro vector was deleted P_{CMV} fragment through treatment of BglII and NheI, pCP vector was constructed through insertion of *Mlu*I-*Hind*III fragment containing P_{CMV} and 5' UTR sequence separated from the pCN vector prepared in example 1 into the pCI-puro vector(see FIG 9a).

On the other hand, pCIII-gag/pol vector was constructed through insertion of the *gag*-*pol* fragment into the pCII vector(Invitrogen), wherein the *gag-pol* fragment originated from MLV(murine leukemia virus) was amplified by PCR, using the conventional gag-pol expression vector, pHIT60(Cannon et al., J. Virol., 70:8234-8240, 1996), as template and the synthetic polynucleotides described by SEQ ID NO: 15 and NO: 16 as primers.

And then, the *gag*-*pol* fragment of the above pCII-gag/pol vector was inserted into the *Mlu*I-*Hind*III site of the above pCP vector, constructing the gag-pol expression vector, pCP-gag/pol vector.

### 2) pCH-env vector construction

The pCH-env vector, which contained hygromycin gene as marker for eukaryote and enhancer/promoter and 5' UTR sequence of HCMV IE gene as regulator, was constructed:

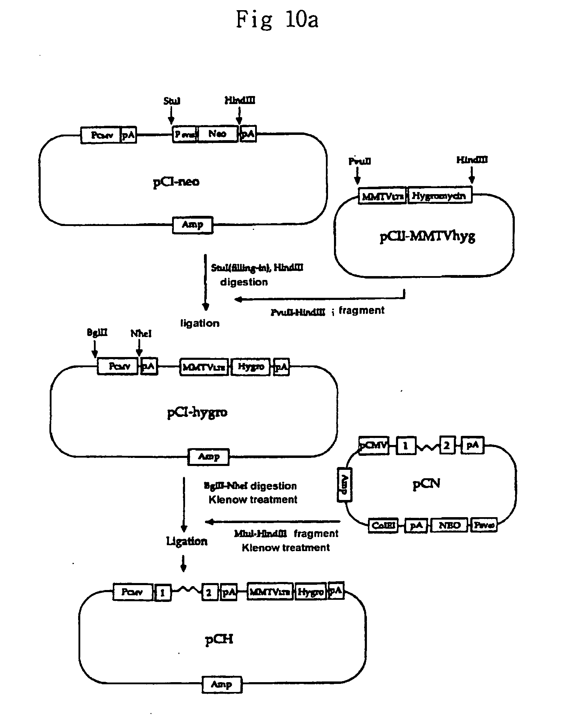

First, neo gene was deleted from the pCI-neo vector(Promega) and then pCI-hygro vector was constructed through insertion of DNA fragment containing hygromycin gene and MMTV(mousse mammary tumor virus) LTR(long terminal repeats) separated from pCII-MMTVhyg vector into the resulting vector(see FIG 5a). After the above pCI-hygro vector was deleted P_{CMV} fragment through treatment of BglII and NheI, pCH vector was constructed through insertion of *Mlu*I-*Hind*III fragment containing P_{CMV} and 5' UTR sequence separated from the pCN vector prepared in example 1 into the pCI-hygro vector(see FIG 10a).

On the other hand, pCH-env vector was constructed through insertion of the *env* fragment into the pCII vector(Invitrogen), wherein the *env* fragment originated from MLV(murine leukemia virus) was amplified by PCR, using the conventional env expression vector, pHIT456(Cannon et al., J. Virol., 70:8234-8240, 1996), as template and the synthetic polynucleotides described by SEQ ID NO: 17 and NO: 18 as primers.

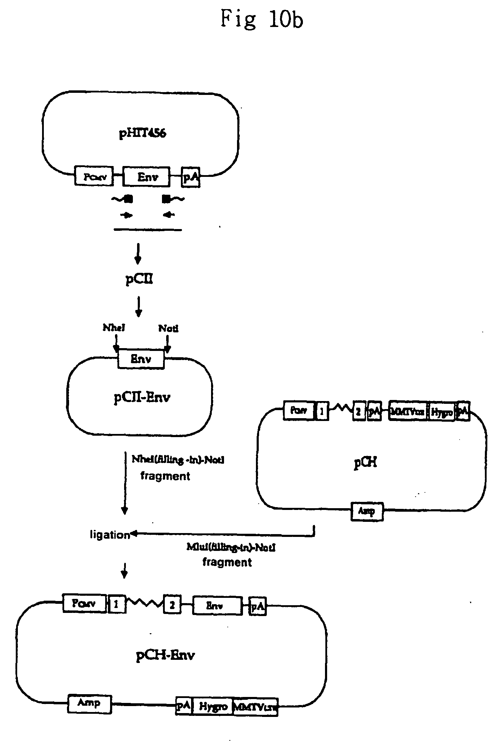

And then, the *env* fragment of the above pCII-env vector was inserted into the *Mlu*I*-Not*I site of the above pCH vector, constructing the env expression vector, pCH-env vector(see FIG 10b).

### 3) Examination of packaging efficiency

To compare the packaging efficiency of the pCP-gag/pol vector and pCH-env vector prepared above with that of conventional packaging vectors, after retroviral MFC-CAT vector(Kim et al., J. Virol., 72:999-1004,1998), together with pCP-gag/pol and pCH-env vectors, were transfected to 293T cells and the transfected cells were cultured for 48 hours, cell-free viral supernatants were obtained by filtrating the cultured medium with 0.45-µm filter.

And then, after the virus were transfected to NIH3T3 cells(ATCC CRL 1658) and the transfected cells were cultured for 48 hours, the efficiency of the retroviral packaging was determined by measurement of CAT activity of the cytosolic protein extract.

As a result, as shown in table 2, it was found that the packaging efficiency of the pCP-gag/pol and pCH-env vector of this invention was increased by about 20 % in comparison with that of the conventional vectors. This suggests that further insertion of the 5' UTR sequence besides the enhancer/promoter sequence help the recombinant vectors to be useful as multipurpose eukaryotic gene expression vectors.

**Table 2**

| Effect of HCMV IE 5'UTR on packaging efficiency | |
|---|---|
| Packaging vector | CAT activity(%) |
| PHIT456, pHIT60 | 50 +/-10 |
| pCP-gag/pol, pCH-env | 60 +/-5 |

### Example 12: Comparison levels the gene expression from pCN, pEF and pCEF vector in cell-culture system.

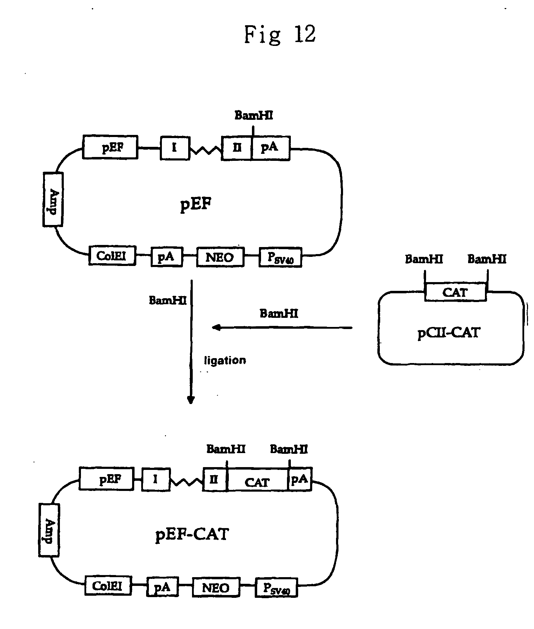

pEF-CAT vector was constructed by inserting CAT fragment into *Hind*III/*Xba*I site of pEF vector (see FIG 12).

pCN-CAT and pEF-CAT were transfected to HeLa cells. 2 days later, CAT activities were measured to compare the levels of gene expression. The gene experssion level of pCMV was set to 1 and those of others were normalized to it. As show in Table 3, in transient transfection system, where gene expression was derived from episomal plasmid not from chromosome, the P_{CMV} drived higher levels of gene expression than pEF did.

In addition, to compare the gene expression level from pCEF with others, pCEF-CAT vector was constructed by insertion of CAT fragment into *Bam*HI site of pCEF. Plasmids pCEF-CAT, pCN-CAT and pEF-CAT were transfected to 293T cells, and CAT activities were measured 48 hours after transfection.

**Table 3**

| Vector | CAT activity |
|---|---|
| pCN-CAT | 7.0 +/-2.0 |
| pEF-CAT | 1.0 |
| PCEF-CAT | 12 +/-1.5 |

As shown in table 3, the CAT activity of the chimeric vector was about 10 fold, and 2-fold higher than that of pEF-CAT, and pCN-CAT, respectively.

### Example 13: Time course of gene expression continuous expression system

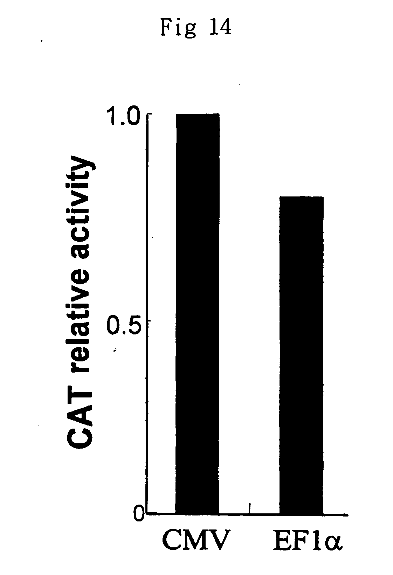

To examine how long and how much the CAT gene could be expressed in continuous culture system, the stable transfected cell lines were generated. Hela cells were transected with pCN-CAT or pEF-CAT and selected with G418. After G418 resistant cells were obtained, CAT activities from each cell line were compared. As shown in Fig.14 the CAT activity of transfected cells with pEF-CAT was close to that of trasfected cells with pCN-CAT. This result suggested that once integrated into the chromosome, the activity of P_{CMV} decreased whereas the activity of P_{EF} was maintained.

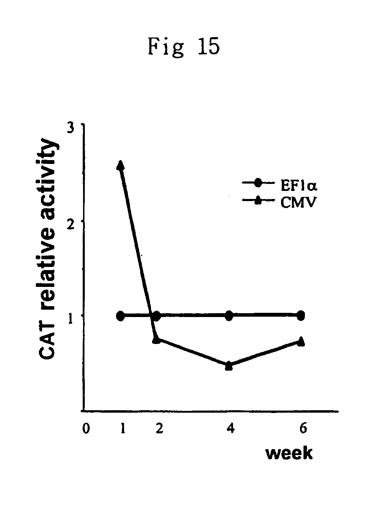

To confirm the above results, the CAT activities from cell lines stably transfected with pCN-CAT or pEF-CAT were measured for 6 weeks as a result, at the beginning, the CAT activity from HCMV promoter was 3 folds higher than that from EF1α promoter. However, 2 weeks later, the CAT activity from HCMV promoter lowered than that from EF1α promoter(see FIG 15). These results confirmed that the level of gene expression derived from P_{CMV} decreased after integrated into the chromosome.

### Example 14: Comparison the levels of gene expression in subclones.

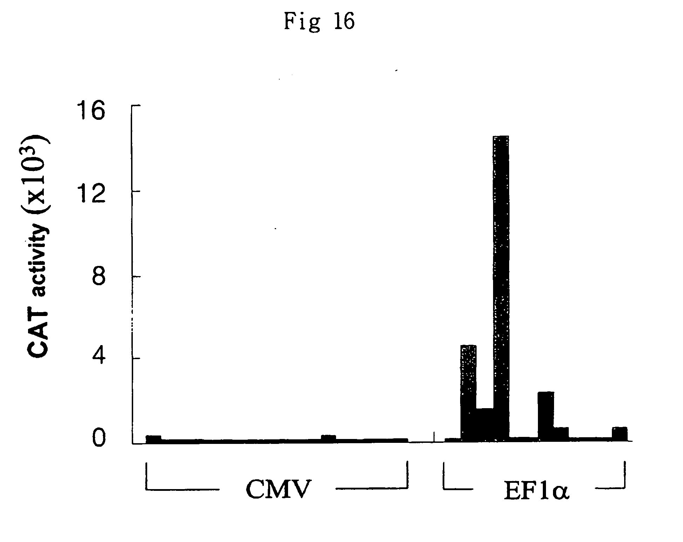

The activities from cell population represent the mean value of the heterogeneous members. However, to use them for industrial purpose, subclones should be characterized. Thus, to examine properties of each subclone, ring cloning method was performed to isolate subclone from population. 25 subclones were isolated from cells transfected with pCN-CAT, and 13 subclones were obtained from cells transfected with pEF-CAT. The results showed that 3 of 25 subclones with PCMV produced significantly high levels of gene expression, whereas most of the remains did not produce CAT protein(see FIG 16). To summarize, most clones derived from stably transfected cells with P_{CMV} seemed to be inactive.

In contrast, 7 of 13 subclones with P_{EF} produced significantly high levels of gene expression. These results suggested that in continuous culture system promoter derived from cellular gene, like EF1α could drive more stable and efficient gene expression than viral promoter P_{CMV} could.

### Example 15 : expression level of VEGF

### 1) VEGF expression in vivo

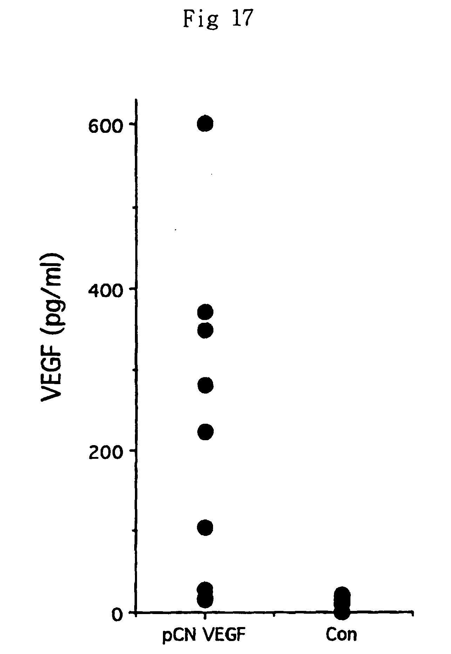

To examine the level of VEGF expression in vivo, the plasmid was injected into anterior tibialis of 2-week-old BALV/C mouse. Two days after injection ELISA was performed using whole protein extracted from the injected muscle. As shown in Fig 17, the level of VEGF expressed in the muscle was significantly high.

### 2) Comparison the level of VEGF expression from CDNA3.1-VEGF165, pCN-VEGF165, pEF-VEGF165 and pCEF-VEGF165 in vivo.

To compare the level of gene expression in vivo, 100µg of plasmids pCDNA3.1-VEGF165, pCN-VEGF165, pEF-VEGF165 and pCEF-VEGF165, were injected into anterior tibialis of 4-week-old BALV/C mouse. 2days later, ELISA was performed with protein extracts from the injected muscle. The results showed that the VEGF expression lefel from pCEF was 9-fold, and 3-fold higher than that from pEF, and pCN, respectively(Fig.17).

### Example 16: Comparison the level of gene expression from pCK and zip in vivo

### 1) Construction of pCK-CAT and pCP-CAT

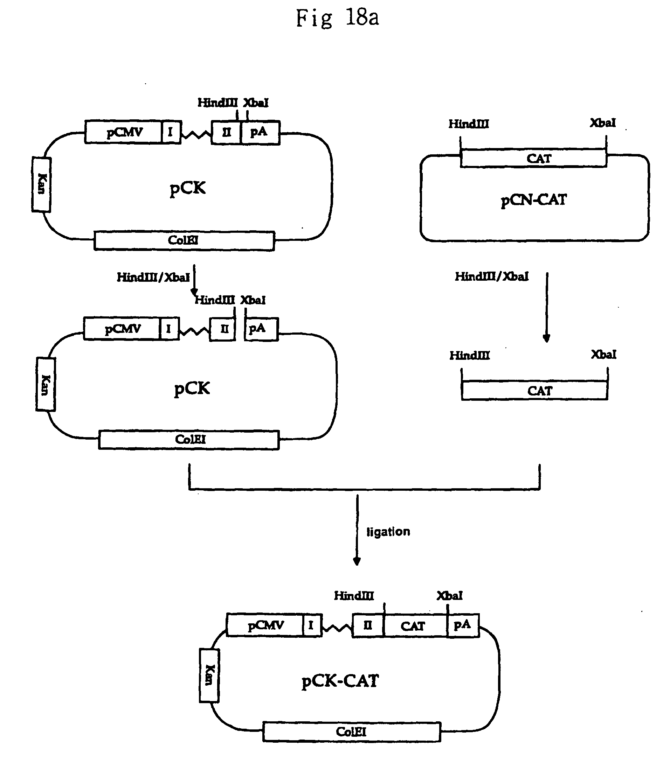

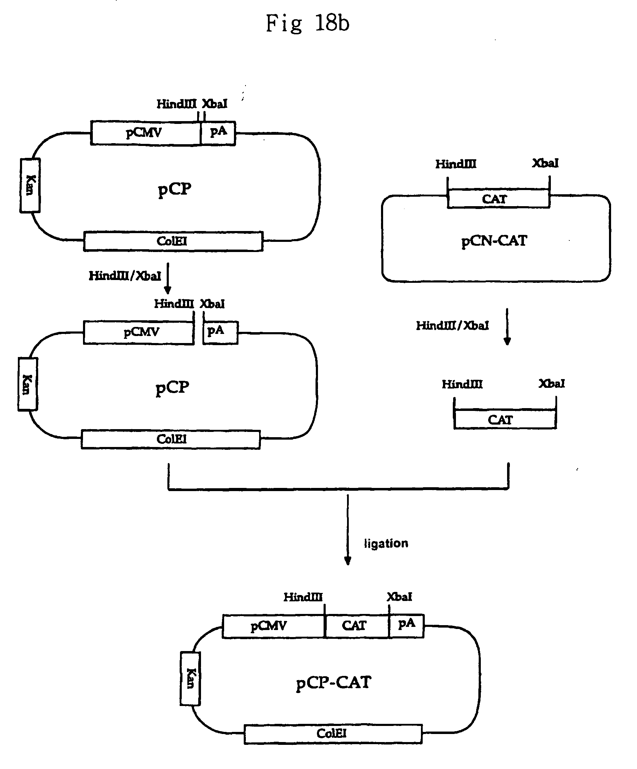

To compare the levels of gene expression from pCK with that from pCP which lacks 5' UTR of HCMV IE gene, pCK-CAT and pCP-CAT were constructed by insertion of CAT reporter gene into HindIII/XbaI site of pCK and pCP vector respectively(see FIG 18).

### 2) Comparison the level of gene expression from pCK and pCP in vitro

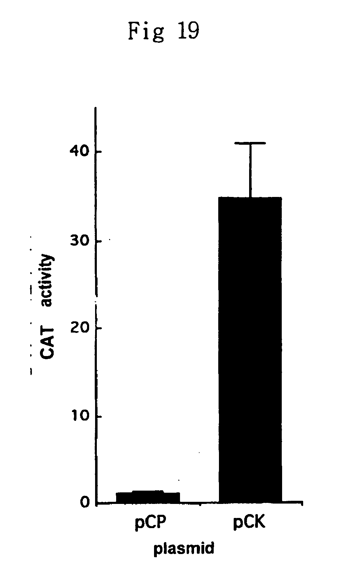

pCK-CAT and pCP-CAT were transfected to C2C12 cells, and the level of CAT activity was measured 2days later. As shown in the result, CAT activity from pCK vector was 30 fold higher than that from pCP(Fig.19).

### 3) Comparison the level of VEGF expression from pCK-VEGF165 and pCP-VEGF165

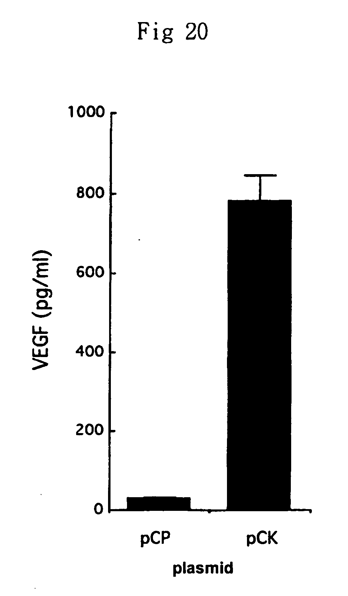

pCK-VEGF and pCP-VEGF were used to transfect C2C12 cells(ATCC CRL-1772) and the levels of VEGF expression were measured 2days later. As a result, it was noted that the expression level from pCK-VEGF was 30-fold higher than that from pCP(see FIG 20).

### Example 17: Comparison the levels of gene expression from pCK and pCP in vivo

### 1) Comparison of pCP-CAT and pCK-CAT in vivo

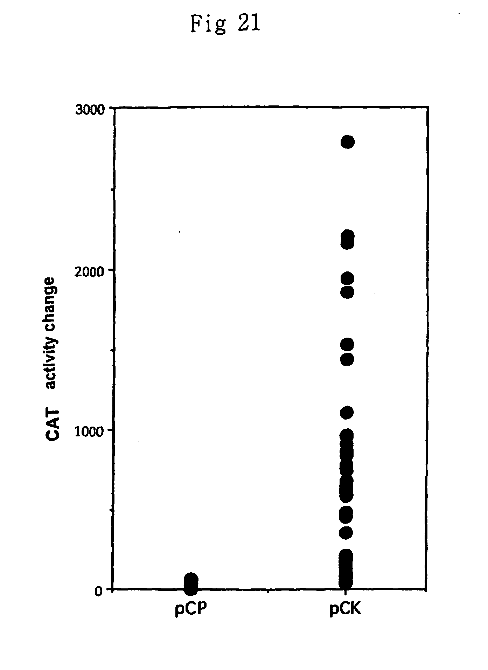

100µg of pCK-CAT and pCP-CAT were injected into anterior tibialis of 2-4-week-old Balb/C male mouse. 48 hrs later, the CAT activities were measured. As shown in Fig21, gene expression level from pCK-CAT was 30 fold higher than that form pCP-CAT(see FIG 21)

### 2) Comparison of pCP-VEGF and pCK-VEGF in vivo

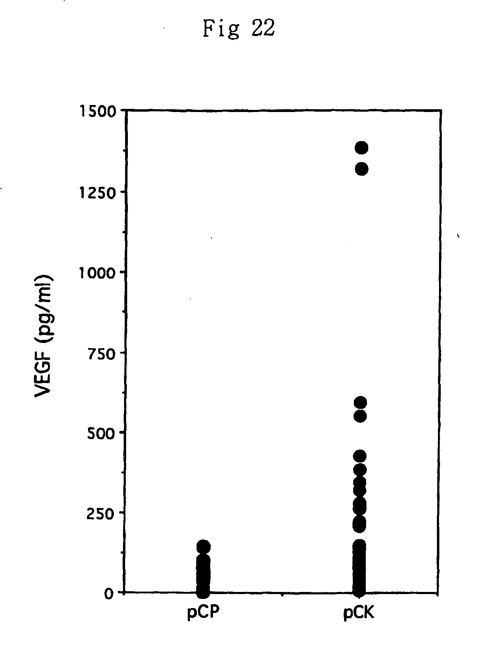

In order to investigate the feasibility of using pCK-VEGF165 vector for naked DNA gene therapy, pCK-VEGF165 and pCP-VEGF165 were injected into anterior tibialis of 2-4-week-old Balb/C male mouse and then ELISA(R&D systems: n=41) was performed using the protein extracts from injected mouse muscle. Particulary, the injection was performed using insulin syringe with 1 mg/ml of DNA dissolved in PBS, wherein DNA was isolated using Endotoxin-free column(Qiagen, Inc.). As shown in the results, pCK-VEGF produced higher levels of gene expression than pCP-VEGF165 in vitro. These results suggests that pCK vector can be used in naked DNA gene therapy(see FIG 22).

### Example 18: Time-course of gene expression

### 1) Time course of CAT production level in vivo

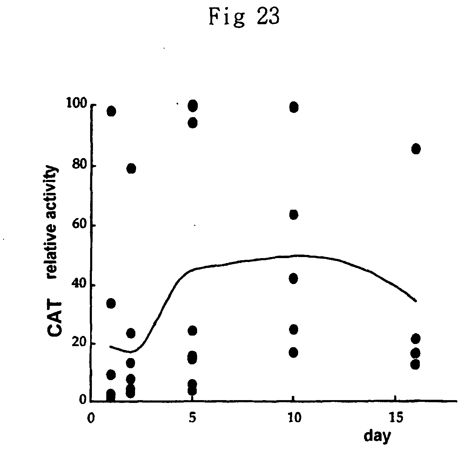

In order to obtain the pharmaceutical kinetics of CAT production in vivo, 100 µg of pCK-CAT vector was injected into anterior tibialis of 2-4-week-old Balb/C male mouse, and the CAT activity was measured for 2 weeks using 9-15 head of mouse. As show in the result, The CAT expression from pCK-CAT vector continued for 2 weeks after injection(see FIG 23).

### 2) Time-course of VEGF165 expression in vivo

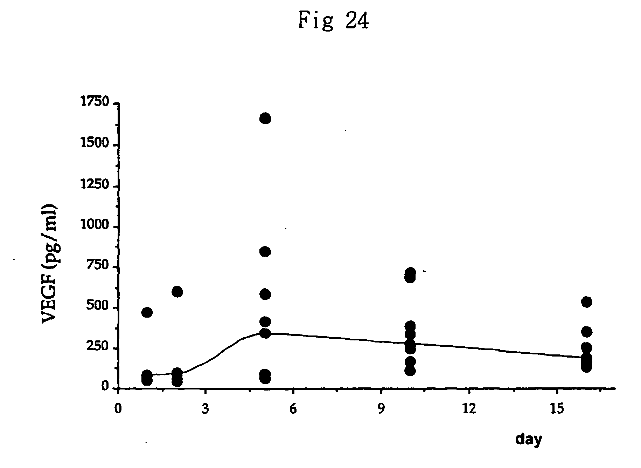

To know kinetics of VEGF expression in vivo, 100 µg of pCK-VEGF165 vector was injected into anterior tibialis of 2-4-week-old Balb/C male mouse and the changes of VEGF165 level were measured for 2 weeks using 12 head of mouse. As shown in Fig 24, the VEGF165 gene expression from pCK-VEGF vector was continued for 2 weeks after injection(see FIG 24).

### Example 19: Dose-dependency of gene expression

### 1) Dose-dependency of CAT expression.

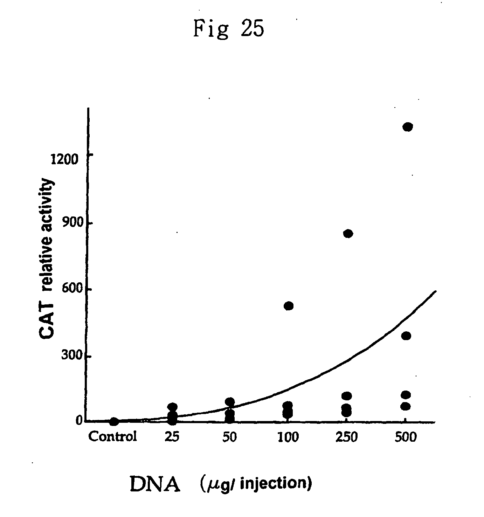

To test the effects of plasmid concentration on the expression level, 2-4-week-old Balb/C male mouse were injected with increasing doses of pCK-CAT, proteins extracted from the injected site 2days later and analyzed. The results showed that the amount of injected pCK-CAT was closely related with level of gene expression(see FIG 25).

### 2) Dose-dependency of VEGF expression in vivo

To test the effects of plasmid concentrations on the expression level, 2-4-week-old Balb/C male mouse were injected with increasing doses of pCK-VEGF. 2 days after injection, the mouse was killed and whole protein of the injected region was extracted to be analyzed by ELISA(n=8-10). The results, it was confirmed above observation that the amount of injected pCK-VEGF was closely related with the level of gene expression(see FIG 25).

### Example 20: Acute toxicity test by parenteral administration to rat mouse

Acute toxicity test was performed using 4-week-old Balb/C SPF mouse. mice were devided by 5 groups and each group of mice were injected with increasing doses of pCK-VEGF165 at the interval of 0.5 upto maximum concentration of 50mg/kg. Plasmids were dissolved in isotonic water, and the injected volume was 200µl.

4-week-old SD SPF, rats were also divided by 5 groups, and each group of rats were injected with increasing doses of pCK-VEGF165 at the interval of 0.5 upto maximum concentration of 200mg/kg. Plasmids were dissolved in isotonic water, and the injected volume was 500µl.

Each group was composed of 5 head of mice, and individuals with standard weight were distributed evenly, The DNA solution was administrated using insulin injector into anterior tibialis of the mouse and rat. An equal amount of PBS was administrated to control group, a week after the administration, death, clinical diagnosis and change of weight of the animals are observed. Then, the animals were autopsied to observe the possible disorders of internal organs.

In every animals tested, any disorder thought to induced by administration of plasmids expressing VEGF was not found. Consequently, the minimal lethal dose of the pCK-VEGF was regarded as 50 mg/kg for mouse and 200 mg/kg for rat.

### INDUSTRIAL APPLICABILITY

As described above, the present invention provides efficient eukaryotic gene expression vectors capable of driving high levels of gene expression in various eukaryotes. The specific properties of vectors of this invention as follows:
1. To construct the vectors using HCMV IE regulatory region, promoter as well as entire 5'UTR(exon 1, intron A, partial exon 2) are used to drive high levels of gene expression in various eukaryotic cells.
2. The vectors with promoter and 5'UTR region of HCMV IE gene can drive strong gene expression in transiently transfected animal cells.
3. Since the vectors with promoter and 5'UTR region of HCMV IE gene can drive high levels of gene expression in vivo, they can be utilized for naked DNA gene therapy.

Those skilled in the art will appreciate that the conceptions and specific embodiments disclosed in the foregoing description may be readily utilized as a basis for modifying or designing other embodiments for carrying out the same purposes of the present invention.

## Claims

1. A enkaryotic expression vector comprising a transcription regulatory element operable linked to a heterologous coding sequence, the transcription regulatory element consisting of the nucleotide sequence of SEQ ID NO: 3.

2. The vector of claim 1, further comprising:
a multi-cloning site;
a polyA signal for transcriptional termination;
a selectable marker gene; and
a ColE1 origin of replication.

3. The vector of claim 2, wherein the selectable marker gene is an ampicillin resistance gene.

4. The vector of claim 2, wherein the selectable marker gene is a kanamycin resistance gene.

5. The vector of claim 4, wherein said vector is 3.7 kb in size.

6. The vector of any one of claims 1 to 5 further comprising a useful foreign gene within the multi-cloning site.

7. The vector of claim 6, wherein the foreign gene is a cDNA encoding a human vascular endothelial growth factor (VEGF).

8. An E.coli cell transformed with the eukaryotic expression vector of any of claims 1 to 7.

9. An expression vector comprising the transcription regulatory element of claim and a gag-pol gene of murine leukemia virus (MLV).

10. An expression vector comprising the transcription regulatory element of claim 1 and an env gene of MLV.

## Patentansprüche

1. Eukaryotischer Expressionsvektor, umfassend ein Transkriptionsregulationselement, das operabel an eine heterologe Kodiersequenz gebunden ist, wobei das Transkriptionsregulationselement aus der Nucleotidsequenz aus Seq.-ID Nr. 3 besteht.

2. Vektor nach Anspruch 1, weiters umfassend:
eine Multiklonierungsstelle;
ein PolyA-Signal für die Transkriptionstermination;
ein selektierbares Marker-Gen; und
einen ColE1-Replikationsstartpunkt.

3. Vektor nach Anspruch 2, worin das selektierbare Markergen ein Ampicillin-Resistenz-Gen ist.

4. Vektor nach Anspruch 2, worin das selektierbare Markergen ein Kanamycin-Resistenz-Gen ist.

5. Vektor nach Anspruch 4, worin der Vektor eine Größe von 3,7 kb aufweist.

6. Vektor nach einem der Ansprüche 1 bis 5, weiters umfassend ein nützliches fremdes Gen innerhalb der Multiklonierungsstelle.

7. Vektor nach Anspruch 6, worin das fremde Gen eine cDNA ist, die für einen menschlichen Gefäßendothel-Wachstumsfaktor (VEGF) kodiert.

8. E.-coli-Zelle, die mit dem eukaryotischen Expressionsvektor nach einem der Ansprüche 1 bis 7 transformiert ist.

9. Expressionsvektor, umfassend das Transkriptionsregulationselement nach Anspruch 1 und ein gag-pol-Gen des murinen Leukämie-Virus (MLV).

10. Expressionsvektor, umfassend das Transkriptionsregulationselement nach Anspruch 1 und ein env-Gen von MLV.

## Revendications

1. Vecteur d'expression eucaryote comprenant un élément régulateur transcriptionnel fonctionnellement lié à une séquence de codage hétérologue, l'élément régulateur transcriptionnel consistant en séquence nucléotide de SEQ ID NO: 3.

2. Vecteur selon la revendication 1, comprenant en outre:
un site de multiclonage;
un signal polyA pour la terminaison transcriptionnelle;
un gène de marqueur sélectionnable; et
une origine ColE1 de réplication.

3. Vecteur selon la revendication 2, dans lequel le gène de marqueur sélectionnable est un gène de résistance à l'ampicilline.

4. Vecteur selon la revendication 2, dans lequel le gène de marqueur sélectionnable est un gène de résistance à la kanamycine.

5. Vecteur selon la revendication 4, dans lequel ledit vecteur a une taille de 3,7 kb.

6. Vecteur selon l'une des revendications 1 à 5, comprenant en outre un gène étranger utile dans le site de multiclonage.

7. Vecteur selon la revendication 6, dans lequel le gène étranger est un ADNc codant pour un facteur de croissance endothéliale vasculaire humain (VEGF).

8. Cellule E.Coli transformée avec un vecteur d'expression eucaryotique selon l'une quelconque des revendications 1 à 7.

9. Vecteur d'expression comprenant l'élément régulateur transcriptionnel selon la revendication 1 et un gène gag-pol du virus de leucémie murine (MLV).

10. Vecteur d'expression comprenant l'élément régulatoire transcriptionnel selon la revendication 1 et un gène env de MLV.


### SEQUENCE LISTING

<110> VIROMED LIMITED
<120> High efficiency mammalian gene expression vectors that contain exogenous promoter and entire 5' untranslated region in the upstream from start codon for inherent gene as transcription regulatory site
<160> 18
<170> KOPATIN 1.0
<210>
   <211> 31
   <212> DNA
   <213> Artificial Sequence
<220>
   <223> CMV pro 5
<220>
   <221> misc_feature
   <222> (1)..(6)
   <223> Mlu I restriction site
<400> 1
   acgcgttgac attgattatt g 21
<210> 2
   <211> 21
   <212> DNA
   <213> Artificial Sequence
<220>
   <223> CMV exon 2,3
<220>
   <221> misc_feature
   <222> (1)..(6)
   <223> Hind III restriction site
<400> 2
   aagcttcgtg tcaaggacgg t 21
<210> 3
   <211> 1567
   <212> DNA
   <213> Human cytomegalovirus
<220>
   <221> promoter
   <222> (1)..(599)
   <223> promoter for human cytomegalovirus
<220>
   <221> exon
   <222> (600)..(720)
   <223> exon 1
<220>
   <221> intron
   <222> (721)..(1547)
   <223> intron A
<220>
   <221> exon
   <222> (1548)..(1564)
   <223> a part of exon 2
<400> 3
   tgacattgat tattgactag ttattaatag taatcaatta cggggtcatt agttcatagc 60
   ccatatatgg agttacgcgt tacataactt acggtaaatg gcccgcctgg ctgaccgccc 120
   aacgaccccc gcccattgac gtcaataatg acgtatgttc ccatagtaac gccaataggg 180
   actttccatt gacgtcaatg ggtggagtat ttacggtaaa ctgcccactt ggcagtacat 240
   caagtgtatc atatgccaag tacgccccct attgacgtca acgacggtaa atggcccgcc 300
   tggcattatg cccagtacat gaccttatgg gactttccta cttggcagta catctacgta 360
   ttagtcatcg ctattaccat ggtgatgcgg ttttggcagt acatcaatgg gcgtggatag 420
   cggtttgact cacggggatt tccaagtctc caccccattg acgtcaatgg gagtttgttt 480
   tggcaccaaa atcaacggga ctttccaaaa tgtcgtaaca actccgcccc attgacgcaa 540
   atgggcggta ggcgtgtacg gtgggaggtc tatataagca gagctcgttt agtgaaccgt 600
   cagatcgcct ggagacgcca tccacgctgt tttgacctcc atagaagaca ccgggaccga 660
   tccagcctcc gcggccggga acggtgcatt ggaacgcgga ttccccgtgc caagagtgac 720
   gtaagtaccg cctatagagt ctataggccc acccccttgg cttcttatgc atgctatact 780
   gtttttggct tggggtctat acacccccgc ttcctcatgt tataggtgat ggtatagctt 840
   agcctatagg tgtgggttat tgaccattat tgaccactcc cctattggtg acgatacttt 900
   ccattactaa tccataacat ggctctttgc cacaactctc tttattggct atatgccaat 960
   acactgtcct tcagagactg acacggactc tgtattttta caggatgggg tctcatttat 1020
   tatttacaaa ttcacatata caacaccacc gtccccagtg cccgcagttt ttattaaaca 1080
   taacgtggga tctccacgcg aatctcgggt acgtgttccg gacatgggct cttctccggt 1140
   agcggcggag cttctacatc cgagccctgc tcccatgcct ccagcgactc atggtcgctc 1200
   ggcagctcct tgctcctaac agtggaggcc agacttaggc acagcacgat gcccaccacc 1260
   accagtgtgc cgcacaaggc cgtggcggta gggtatgtgt ctgaaaatga gctcggggag 1320
   cgggcttgca ccgctgacgc atttggaaga cttaaggcag cggcagaaga agatgcaggc 1380
   agctgagttg ttgtgttctg ataagagtca gaggtaactc ccgttacggt gctgttaacg 1440
   gtggagggca gtgtagtctg agcagtactc gttgctgccg cgcgcgccac cagacataat 1500
   agctgacaga ctaacagact gttcctttcc atgggtcttt tctgcagtca ccgtccttga 1560
   cacgatg 1567
<210> 4
   <211> 36
   <212> DNA
   <213> Artificial Sequence
<220>
   <223> PRIMER
<220>
   <221> misc_feature
   <222> (1)..(6)
   <223> Mlu I restrition site
<400> 4
   acgcgtggca attgaaccgg tgcctagaga aggtgg 36
<210> 5
   <211> 36
   <212> DNA
   <213> Artificial Sequence
<220>
   <223> PRIMER
<220>
   <221> misc_feature
   <222> (1)..(6)
   <223> Nhe I restriction site
<400> 5
   gctagctttg gcttttaggg gtagttttca cgacac 36
<210> 6
   <211> 1139
   <212> DNA
   <213> Homo sapiens
<220>
   <221> promoter
   <222> (1)..(137)
   <223> promoter for human EF1-alpha gene
<220>
   <221> exon
   <222> (138)..(158)
   <223> exon 1
<220>
   <221> intron
   <222> (159)..(1104)
   <223> intron A
<220>
   <221> exon
   <222> (1105)..(1136)
   <223> a part of exon 2
<400> 6
   ggcaattgaa ccggtgccta gagaaggtgg cgcggggtaa actgggaaag tgatgtcgtg 60
   tactggctcc gcctttttcc cgagggtggg ggagaaccgt atataagtgc agtagtcgcc 120
   gtgaacgttc tttttcgcaa cgggtttgcc gccagaacac aggtaagtgc cgtgtgtggt 180
   tcccgcgggc ctggcctctt tacgggttat ggcccttgcg tgccttgaat tacttccacg 240
   cccctggctg cagtacgtga ttcttgatcc cgagcttcgg gttggaagtg ggtgggagag 300
   ttcgaggcct tgcgcttaag gagccccttc gcctcgtgct tgagttgagg cctggcctgg 360
   gcgctggggc cgccgcgtgc gaatctggtg gcaccttcgc gcctgtctcg ctgctttcga 420
   taagtctcta gccatttaaa atttttgatg acctgctgcg acgctttttt tctggcaaga 480
   tagtcttgta aatgcgggcc aagatctgca cactggtatt tcggtttttg gggccgcggg 540
   cggcgacggg gcccgtgcgt cccagcgcac atgttcggcg aggcggggcc tgcgagcgcg 600
   gccaccgaga atcggacggg ggtagtctca agctggccgg cctgctctgg tgcctggcct 660
   cgcgccgccg tgtatcgccc cgccctgggc ggcaaggctg gcccggtcgg caccagttgc 720
   gtgagcggaa agatggccgc ttcccggccc tgctgcaggg agctcaaaat ggaggacgcg 780
   gcgctcggga gagcgggcgg gtgagtcacc cacacaaagg aaaagggcct ttccgtcctc 840
   agccgtcgct tcatgtgact ccacggagta ccgggcgccg tccaggcacc tcgattagtt 900
   ctcgagcttt tggagtacgt cgtctttagg ttggggggag gggttttatg cgatggagtt 960
   tccccacact gagtgggtgg agactgaagt taggccagct tggcacttga tgtaattctc 1020
   cttggaattt gccctttttg agtttggatc ttggttcatt ctcaagcctc agacagtggt 1080
   tcaaagtttt tttcttccat ttcaggtgtc gtgaaaacta cccctaaaag ccaaaaatg 1139
<210> 7
   <211> 27
   <212> DNA
   <213> Artificial Sequence
<220>
   <223> CMV 55
<220>
   <221> misc_feature
   <222> (1)..(5)
   <223> Bgl II restrition site
<220>
   <221> misc_feature
   <222> (7)..(12)
   <223> Mlu I restrition site
<400> 7
   agatctacgc gttgacattg attattg 27
<210> 8
   <211> 25
   <212> DNA
   <213> Artificial Sequence
<220>
   <223> CMV 53
<220>
   <221> misc_feature
   <222> (1)..(6)
   <223> Hpa I restrition site
<400> 8
   gttaactcta taggcggtac ttacg 25
<210> 9
   <211> 21
   <212> DNA
   <213> Artificial Sequence
<220>
   <223> PRIMER
<220>
   <221> misc-feature
   <222> (1)..(6)
   <223> Mlu I restrition site
<400> 9
   acgcgttgac attgattatt g 21
<210> 10
   <211> 30
   <212> DNA
   <213> Artificial Sequence
<220>
   <223> PRIMER
<220>
   <221> misc_feature
   <222> (1)..(6)
   <223> Sal 1 restrition site
<400> 10
   gtcgactccc cagcatgcct gctattgtct 30
<210> 11
   <211> 18
   <212> DNA
   <213> Artificial Sequence
<220>
   <223> SENSE PRIMER
<400> 11
   atgaactttc tgctgtct 18
<210> 12
   <211> 24
   <212> DNA
   <213> Artificial Sequence
<220>
   <223> ANTISENSE PRIMER
<400> 12
   tcacctcggc ttgtcacatt tttc 24
<210> 13
   <211> 24
   <212> DNA
   <213> Artificial Sequence
<220>
   <223> PRIMER
<220>
   <221> misc_feature
   <222> (1)..(6)
   <223> Hind III restrition site
<400> 13
   aagcttatga actttctgct gtct 24
<210> 14
   <211> 30
   <212> DNA
   <213> Artificial Sequence
<220>
   <223> PRIMER
<220>
   <221> misc_feature
   <222> (1)..(6)
   <223> Xba 1 restrition site
<400> 14
   tctagatcac cgcctcggct tgtcacatca 30
<210> 15
   <211> 24
   <212> DNA
   <213> Artificial Sequence
<220>
   <223> gp5
<220>
   <221> misc_feature
   <222> (1)..(6)
   <223> Nhe I restrition site
<400> 15
   gctagcgcaa ccctgggaga cgtc 24
<210> 16
   <211> 26
   <212> DNA
   <213> Artificial Sequence
<220>
   <223> gp3
<220>
   <221> misc_feature
   <222> (1)..(8)
   <223> Not I restrition site
<400> 16
   gcggccgcac tgacccctct gagcat 26
<210> 17
   <211> 24
   <212> DNA
   <213> Artificial Sequence
<220>
   <223> env 5
<220>
   <221> misc_feature
   <222> (1)..(6)
   <223> Nhe I restrition site
<400> 17
   gctagcgcca ccatggcgcg ttca 24
<210> 18
   <211> 26
   <212> DNA
   <213> Artificial Sequence
<220>
   <223> env 3
<220>
   <221> misc_feature
   <222> (1)..(8)
   <223> Not I restrition site
<400> 18
   gcggccgcga tatctcatgg ctcgta 26